# DEMANDE DE BREVET EUROPEEN

(11) **EP 2 037 276 A2**
(43) Date de publication de la demande: **18.03.2009**
(21) Numéro de dépôt: 08019610.8
(22) Date de dépôt: 27.08.2002
(51) Int. Cl.: G01N 33/68, G01N 33/50, C07K 17/00

(54) **Test de l'immunité cellulaire par des peptides fixés sur support solide**

(30) Priorité: 27.08.2001 FR 0111136
(62) Demande divisionnaire de: 02774883.9
(71) Demandeur: INSTITUT NATIONAL DE LA SANTE ET DE LA RECHERCHE MEDICALE (INSERM), 75013 Paris (FR)
(72) Inventeur: Gahery-Segard, Hanne, 75014 Paris (FR); Guillet, Jean-Gérard, 75009 Paris (FR)
(74) Mandataire: Vialle-Presles, Marie José

(57) **Abrégé**

Support solide sur lequel est (sont) fixé(s) un ou plusieurs peptide(s) constituant un (des) épitope(s) T CD4+ et/ou T CD8+ d'un antigène.

## Description

L'invention est relative à de nouveaux moyens de détection de l'immunité cellulaire.

L'immunité à médiation cellulaire joue un rôle majeur dans la réponse immunitaire vis-à-vis de microorganismes tels que des virus, ainsi que certaines bactéries ou certains protozoaires qui sont capables de se développer à l'intérieur des cellules de l'hôte, échappant ainsi aux anticorps. Elle intervient aussi dans des phénomènes de rejet de greffe et la destruction de tumeurs.

Alors que dans le cas de l'immunité humorale, les effecteurs moléculaires sont les anticorps sécrétés par les lymphocytes B, les effecteurs moléculaires de l'immunité cellulaire sont les récepteurs TcR ancrés dans la membrane cytoplasmique des lymphocytes T.

La reconnaissance d'un épitope T par le TcR implique un mécanisme beaucoup plus complexe que celui qui intervient dans la reconnaissance d'un épitope B par un anticorps. En effet, le TcR ne reconnaîtra le peptide constituant son épitope que si celui-ci est associé à une molécule de classe I ou de classe II du CMH (complexe majeur d'histocompatibilité) à la surface d'une cellule présentatrice. Les TcR des lymphocytes T CD4 et CD8 reconnaissent ainsi les épitopes présentés respectivement par les molécules de classe II et par les molécules de classe I du CMH.

On définit comme « épitope T » d'un antigène, tout fragment peptidique dudit antigène capable d'être reconnu dans le contexte de CMH approprié, par le récepteur TcR d'un lymphocyte T, et d'induire l'activation dudit lymphocyte.

La taille d'un épitope T varie selon la classe de la molécule de CMH présentant le peptide ; les épitopes T CD8⁺ présentés par les molécules du CMH de classe I ont une taille de 8 à 10 acides aminés, alors que les épitopes T CD4⁺ présentés par les molécules du CMH de classe II ont une taille de 13 à 25 acides aminés.

Pour un même antigène et pour une même classe de CMH, la séquence des épitopes varie selon l'allèle du CMH concerné.

Pour de très nombreux antigènes, une grande variété d'épitopes T restreints à différents allèles du CMH de classe II ou du CMH de classe I a été identifiée et les séquences de ces épitopes sont disponibles dans la littérature.

L'interaction TcR-épitope T associé au CMH induit, en présence de divers signaux de co-stimulation qui résultent de l'interaction de molécules en surface des cellules T et de molécules en surface des cellules présentatrices, une stimulation des lymphocytes se traduisant par une multiplication cellulaire aboutissant à des lymphocytes T effecteurs.

Les lymphocytes effecteurs peuvent être regroupés en différentes populations selon leurs fonctions effectrices, qui définissent divers types de réponse immunitaire. Ces populations peuvent être distinguées notamment par leur capacité à sécréter différentes molécules (cytokines, facteurs lytiques) ou combinaisons de molécules particulières. Par exemple, parmi les lymphocytes effecteurs qui peuvent se différencier à partir des cellules CD4⁺ ou CD8⁺, on citera les lymphocytes Th1 ou T1, qui se caractérisent par la sécrétion de cytokines notamment l'interleukine 2 (IL-2) et l'interféron gamma (IFN-γ), et les cellules Th2 ou T2 qui sécrètent des cytokines telles que l'IL-4, l'IL-5, l'IL-6 et l'IL-10.

Contrairement à l'immunité humorale qui n'est détectable d'une façon significative environ 1 mois après un premier contact avec un immunogène, l'immunité à médiation cellulaire apparaît en quelques jours après ce premier contact ; elle persiste ensuite pendant une longue période, sous forme de lymphocytes T mémoire, qui sont capables de proliférer et d'acquérir rapidement des fonctions effectrices lors d'une présentation ultérieure du même immunogène.

L'immunité cellulaire peut donc constituer un bon indicateur, permettant la détection très précoce d'une réponse immunitaire vis-à-vis d'un antigène déterminé, et la détermination du type de réponse immunitaire mise en jeu.

La détection de lymphocytes T effecteurs spécifiques d'antigène est utilisée dans différents laboratoires de recherche, par exemple dans le cadre de la mise au point de vaccins, pour rechercher les épitopes immunisants et évaluer l'intensité et le type de la réponse immunogène.

Une approche de plus en plus souvent utilisée pour évaluer l'immunité cellulaire vis-à-vis d'un antigène consiste à fournir des peptides constituant des épitopes T connus dudit antigène et à analyser la réactivité des lymphocytes T vis-à-vis de ces peptides.

Cette approche peut théoriquement être utilisée pour le diagnostic ou le suivi de pathologies entraînant une réponse à médiation cellulaire. Toutefois, contrairement à l'immunité humorale, que l'on peut détecter facilement *in vitro* par mise en évidence de la formation d'un complexe antigène-anticorps, la détection de l'immunité cellulaire implique la mise en oeuvre de techniques lourdes difficilement utilisables dans le cadre d'analyses de routine.

Il est en effet nécessaire :
1) de présenter aux lymphocytes les épitopes à tester, dans des conditions permettant leur reconnaissance par le TcR et l'activation des lymphocytes ;
2) de détecter les lymphocytes effecteurs activés.

Certaines méthodes développées récemment permettent de simplifier la détection des cellules effectrices. A titre d'exemple, la technique ELISPOT, initialement décrite par VERSTEEGEN et al. (J. Immunol. Methods, 111, 25-29, 1988) permet de détecter et caractériser des lymphocytes T effecteurs spécifiques d'épitopes, présents parmi les cellules du sang périphérique, par détection des facteurs sécrétés par lesdits lymphocytes après re-stimulation par des peptides représentant des épitopes T.

Par exemple, cette technique a été utilisée pour identifier des lymphocytes effecteurs T CD8⁺ spécifiques d'épitopes du virus de la grippe (LALVANI et al., J. Exp. Med. 186, 859-865, 1997) ; pour analyser la réponse T CD8⁺ vis-à-vis de peptides représentant divers épitopes de HIV1 utilisés dans des vaccins (GAHERY-SEGARD et al., J. Virol., 74, 1694-1703, 2000) ; pour déceler la présence d'une infection par *Mycobacterium tuberculosis* par détection de lymphocytes effecteurs T spécifiques d'épitopes de l'antigène ESAT-6 (LALVANI et al., Am. J. Respir. Crit. Care Med., 163, 824-828, 2001).

Les essais décrits dans les publications mentionnées ci-dessus ont été effectués en ajoutant une solution de chacun des peptides à tester à une suspension obtenue par séparation sur gradient de FICOL de cellules monocytaires du sang périphérique (PBMCs) ; on sait en effet que dans ces conditions, ces peptides peuvent être chargés directement par les molécules du CMH et présentés aux lymphocytes.

Or les Inventeurs ont maintenant constaté que, de façon surprenante, la mise en contact des lymphocytes T d'un sujet avec des peptides constituant des épitopes reconnus par lesdits lymphocytes T, fixés sur un support solide, entraînait l'activation de lymphocytes T effecteurs spécifiques desdits épitopes.

La présente invention a pour objet l'utilisation d'un support solide auquel est fixé au moins un peptide constituant un épitope T d'un antigène pour la détection et/ou la caractérisation *in vitro* de l'immunité cellulaire vis-à-vis dudit antigène.

La présente invention a en particulier pour objet un procédé de détection et/ou de caractérisation *in vitro* de la réponse immune cellulaire d'un sujet vis-à-vis d'un antigène, par mise en contact d'un échantillon biologique comprenant des lymphocytes T CD4⁺ et/ou des lymphocytes T CD8⁺ présents dans les cellules monocytaires du sang périphérique (PBMCs) dudit sujet avec un ou plusieurs peptide(s) constituant un (des) épitope(s) T dudit antigène capable(s) d'être présenté(s) par une molécule du CMH dudit sujet et détection des lymphocytes T effecteurs activés par cette mise en contact, lequel procédé est caractérisé en ce que le(s)dit(s) peptide(s) est (sont) fixé(s) sur un support solide.

L'échantillon biologique peut être constitué par une préparation de lymphocytes T CD4⁺ ou CD8⁺ purifiés. Il peut aussi être constitué par une préparation de PBMCs obtenue à partir d'un prélèvement de sang dudit sujet ; avantageusement, on peut également utiliser du sang total.

Selon un mode de mise en oeuvre préféré du procédé conforme à l'invention, au moins 2 peptides constituant 2 épitopes T différents dudit antigène, et dont l'un au moins est capable d'être présenté par une molécule du CMH dudit sujet sont fixés audit support solide.

Selon une disposition avantageuse de ce mode de mise en oeuvre, un mélange desdits peptides est fixé audit support solide.

Selon une autre disposition avantageuse de ce mode de mise en oeuvre, ledit support solide est constitué par une plaque de microtitration, lesdits peptides étant fixés dans au moins 2 puits différents de ladite plaque et l'échantillon biologique est divisé en au moins 2 aliquotes dont chacune est mise en contact avec l'un desdits peptides.

L'utilisation d'un mélange de peptides permet de détecter rapidement l'existence d'une réponse immune à médiation cellulaire vis-à-vis de l'antigène concerné. L'utilisation séparée de différents peptides permet de mieux analyser les composantes de cette réponse.

Dans tous les cas, le(s) peptide(s) utilisé(s) peu(ven)t constituer un (des) épitope(s) T CD4⁺, ou un (des) épitope(s) T CD8⁺. On peut également utiliser simultanément un (des) épitope(s) T CD4⁺, et un (des) épitope(s) T CD8⁺, séparément ou en mélange.

Les autres modalités de mise en oeuvre du procédé conforme à l'invention peuvent être identiques à celles utilisées dans les méthodes de détection *in vitro* de la réponse immune cellulaire connues dans l'art antérieur.

Par exemple, la mise en contact entre les peptides fixés au support solide et l'échantillon biologique à tester s'effectue dans les mêmes conditions que dans les méthodes de l'art antérieur où les peptides sont présents dans la phase liquide. Généralement, cette mise en contact s'effectuera par incubation pendant 5 à 20 heures à 37°C, en présence de 5% de CO₂.

De même, la détection des lymphocytes T effecteurs activés peut s'effectuer par les méthodes classiques.

Avantageusement, on effectuera le dosage des facteurs solubles (cytokines ou facteurs lytiques) sécrétés par ces lymphocytes. Par exemple, le dosage de l'IL-2 ou de l'IFN-γ permet de caractériser une réponse Th1 ou T1, alors que le dosage de l'IL-4, l'IL-5, l'IL-6 ou l'IL-10, permet de caractériser une réponse Th2 ou T2. D'autres facteurs solubles qui caractérisent essentiellement les lymphocytes T CD8⁺ comme les chimiokines (RANTES), ou les molécules à fonction de lyse (comme perforine/granzyme) peuvent également être dosés.

D'autres méthodes de détection des lymphocytes T activés éventuellement utilisables dans le cadre de la présente invention sont par exemple le marquage intracellulaire des cytokines.

Par rapport aux méthodes d'évaluation de l'immunité cellulaire connues dans l'art antérieur, dans lesquelles les peptides à tester sont ajoutés dans la suspension de cellules, le procédé conforme à l'invention permet de simplifier considérablement la procédure d'essai, notamment dans le cas d'analyses impliquant l'utilisation en parallèle de différents peptides, qui s'effectuent classiquement dans les puits d'une plaque de microtitration, ou dans une série de tubes.

En effet, alors que les techniques de l'art antérieur nécessitent d'ajouter aux cellules à tester un peptide ou un mélange de peptides défini dans chacun des puits de la plaque ou dans chaque tube de la série, il est possible, dans le cadre de la mise en oeuvre de la présente invention, d'utiliser des plaques ou des tubes préparés d'avance, chacun des puits desdites plaques ou chacun desdits tubes étant recouvert du peptide ou du mélange de peptides souhaité. Ceci permet de réduire considérablement les manipulations nécessaires, donc de diminuer le temps nécessaire à la mise en oeuvre de l'essai et d'augmenter sa reproductibilité en limitant les causes d'erreur.

En outre, la fixation des peptides sur un support solide permet de stabiliser ceux-ci et de les protéger de la dégradation qui intervient rapidement en milieu liquide. A titre d'illustration, dans le cas de peptides fixés par adsorption sur les puits d'une plaque de microtitration en polystyrène, les peptides conservent leurs propriétés d'activation spécifique des lymphocytes T pendant au moins 24 heures à 20°C, au moins une semaine à 4°C, et au moins 15 jours dans un congélateur à -80°C, alors que des solutions des mêmes peptides perdent leurs propriétés en quelques heures à 37°C.

De plus, la présente invention présente l'avantage de diminuer considérablement les concentrations de peptides utilisés, permettant ainsi une diminùtion très conséquente des coûts.

La présente invention a également pour objet un kit pour la mise en oeuvre d'un procédé conforme à l'invention, comprenant un assortiment de peptides issus d'un même antigène, dont chacun constitue un épitope T dudit antigène, fixés sur un support solide.

Pour la réalisation de kits conformes à l'invention, on peut utiliser des supports solides, et des méthodes de fixation des peptides auxdits supports, de même type que ceux qui sont habituellement utilisés notamment pour les kits de dosage antigène anticorps.

Par exemple, on peut utiliser des supports en matériaux plastiques tels que le polystyrène, le polyéthylène ou le chlorure de polyvinyle, des supports en nitrocellulose, des supports en matériaux à base de silice, tels que le verre, etc.

La fixation des peptides peut s'effectuer par des techniques également connues en elles-mêmes ; le choix des techniques les plus appropriées dépend du support concerné, et des propriétés physicochimiques des peptides choisis.

On peut par exemple synthétiser directement les peptides sur le support, ou fixer à celui-ci des peptides présynthétisés.

Par exemple, la fixation des peptides peut s'effectuer par adsorption. Le support et les conditions de fixation seront bien entendu choisis de manière à éviter la désorption des peptides lors de l'addition de l'échantillon biologique à tester et de l'incubation avec celui-ci.

Avantageusement, les peptides seront fixés au support par liaison covalente, directement ou par l'intermédiaire d'un bras espaceur. Dans ce cas la surface du support peut être activée par l'intermédiaires d'agents tels que les dérivés de carbodiimide, les dérivés de N-hydroxysuccynimide, les dérivés de sulfosuccynimide etc.

On peut également utiliser des peptides biotinylés, fixés sur un support recouvert d'avidine.

Le support peut être sous forme d'un tube ou d'un ensemble de tubes, d'une plaque de microtitration, d'une ou plusieurs bandelettes, de billes, etc.

L'antigène peut être en particulier un micro-organisme infectieux (virus, protozoaire, bactérie), un antigène tumoral, un antigène auto-immun ou un allergène. S'il s'agit d'un micro-organisme, les peptides constituant l'assortiment peuvent être issus de différentes protéines antigéniques de cet organisme.

Le choix des peptides constituant l'assortiment dépend bien entendu de l'antigène concerné, et de l'utilisation précise à laquelle est destiné le kit. Il s'agira de peptides précédemment identifiés comme constituant des épitopes T CD4⁺ ou CD8⁺ de l'antigène concerné.

Avantageusement, cet assortiment comprendra des peptides capables d'être présentés par des allèles différents du CMH. Il peut être constitué de peptides constituant des épitopes T CD4⁺, de peptides constituant des épitopes T CD8⁺, ou d'un mélange de ces deux types d'épitopes.

Les peptides constituant l'assortiment peuvent être mélangés et fixés sous forme de mélange au support solide. Lorsque le support solide est une plaque de microtitration ou un ensemble de tubes, différents peptides ou combinaisons de peptides peuvent également être fixés séparément, chaque peptide ou combinaison de peptide étant fixé à l'intérieur de l'un des puits de la plaque de microtitration, ou de l'un des tubes.

Un kit conforme à l'invention peut également comprendre en outre des moyens de détection des lymphocytes T effecteurs activés. Ces moyens peuvent par être constitués par des réactifs de dosage des facteurs solubles produits par lesdits lymphocytes.

La présente invention peut par exemple être mise en oeuvre, en santé humaine ou animale :
- dans le cadre du dépistage et du suivi de l'évolution d'infections par différents agents pathogènes entraînant une réponse immunitaire cellulaire. A titre d'exemples on citera : des virus tels que les HIV (human immunodeficiency virus) 1 et 2, le FIV (feline immunodeficiency virus), le HTLV-1 (Human T Lymphocyte Virus type I), les virus des différents types d'hépatite, etc ; des parasites tels que *Toxoplasma,* ou *Plasmodium* ; les prions, etc. ;
- dans le cadre du dépistage et du suivi de l'évolution de pathologies d'origines tumorales. A titre d'exemples on citera : le mélanome, le cancer du sein, les leucémies myéloïdes, les cancers associés à une infection virale, comme le cancer du col associé au papilloma virus humain, etc. ;
- dans le cadre du dépistage et du suivi de l'évolution de maladies auto-immunes. A titre d'exemple on citera le diabète de type I, la sclérose en plaques, etc. ;
- dans le cadre du dépistage et du suivi de l'évolution des maladies de type allergique. A titre d'exemple, on citera des allergènes comme les graminées, les pollens, certains médicaments.

Quelle que soit l'utilisation concernée, la présente invention permet, par la détection de l'immunité à médiation cellulaire, un diagnostic très précoce de l'apparition ou de l'évolution d'une pathologie, avant l'apparition des signes cliniques et avant l'apparition de la réponse humorale. Ceci rend possible une décision médicale plus rapide, une meilleure stratégie thérapeutique et de meilleures chances de succès du traitement pour les patients.

La présente invention sera mieux comprise à l'aide du complément de description qui va suivre, qui se réfère à des exemples non-limitatifs illustrant sa mise en oeuvre pour la caractérisation d'une immunité cellulaire spécifique vis-à-vis de différents pathogènes viraux.

### EXEMPLE 1 : FIXATION SUR UN SUPPORT SOLIDE DE PEPTIDES CONSTITUANT DES EPITOPES T.

Différents peptides constituant des épitopes CD8⁺ connus de trois virus différents : le virus HIV-1 (virus de l'immunodéficience humaine), le CMV (cytomegalovirus) et le virus EBV (virus d' Epstein Barr), ont été synthétisés.

Ces peptides sont les suivants :
Virus HIV-1:
   gag 77-85 (SLYNTVATL, HLA A2)
   GAG 260-268 (EIYKRWIIL, HLA A2)
   Nef 82-91 (KAALDLSHFL, HLA A2)
Virus CMV :
   GB 619-628 (FIAGNSAYEYV, HLA A2)
   IEI 378-389(SDEEEAIVAYTL, HLA B18)
Virus EBV :
   EBNA 3A 379-387(RPPIFIRRL, HLA B7)
   EBNA 3C 163-171 (EGGVGWRHV, HLA B44).

Ces peptides ont été fixés au fond des puits d'une plaque en polystyrène, par le protocole suivant.

10 µg de peptide en tampon carbonate ont été déposés dans une plaque de microtitration à 96 puits (plaque en polystyrène, NUNC MAXISORB). La fixation des peptides s'est faite par adsorption pendant 2 heures à température ambiante. Les peptides non-fixés sont éliminés par deux lavages en PBS.

### EXEMPLE 2 : CARACTERISATION D'UNE IMMUNITE CELLULAIRE VIS-A-VIS DE HIV-1

Des PBMCs purifiées à partir du sang d'un patient (HLA-A2) séropositif pour HIV-1 ont été testées comme suit.

### 1) Activation des lymphocytes :

200 µl (10⁵ cellules) de suspension cellulaire dans du milieu de culture classique (RPMI, 10% de SAB) sont déposés dans chacun des puits dans lesquels lequel ont été fixés les peptides de HIV-1, comme décrit à l'Exemple 1 ci-dessus.

A titre de témoin positif, un essai a été effectué par la méthode ELISPOT classique : 200 µl (10⁵ cellules) de suspension des PBMCs du même patient sont déposés dans les puits d'une plaque 96 puits, NUNC (identique à celle sur laquelle ont été fixés les peptides), et mélangées avec 10 µg de peptide gag 77-85 ,GAG 260-268, ou Nef 82-91 en solution dans du milieu de culture classique.

Dans les 2 essais, des PBMCs ont également été déposées dans des puits sans peptides, à titre de contrôle.

Les plaques sont incubées une nuit à 37^{°}C dans un incubateur en présence de 5% de C0₂.

### 2) Sélection des lymphocytes T CD8⁺ activés :

Le lendemain, la présence des cellules CD8⁺ activées est révélée selon le protocole suivant :

Une plaque de 96 puits en nitrocellulose (MILLIPORE) a été recouverte avec 100 µl/puits d'anticorps anti-IFNγ (MABTECH, 1-D1K), dilué à 1 µg/ml dans du tampon carbonate pendant 2 heures à 37°C.

La plaque a été ensuite lavée en PBS et saturée avec du RPMI, SVF 10% à raison de 100 µl/puits, pendant 2 heures à 37°C.

200 µl de suspension cellulaire activée comme décrit en 1) ci-dessus sont déposés dans cette plaque. L'incubation s'effectue pendant 5 heures à 37°C, 5% CO₂.

Après 1. lavage en PBS 1X et 5 lavages en PBS TWEEN 0,05%, on ajoute 100 µl/puits d'anticorps anti-IFN-γ Biotin (MABTECH, 7-B6-1-Biotin) à 1 µg/ml, et on incube pendant de 2 heures à température ambiante.

Les plaques sont lavées 5 fois en PBS-TWEEN 0,05%.

100 µl/puits Extravidin-AKP (SIGMA, n° 2636) dilué au 1/5000 en PBS TWEEN 0,05%, BSA 1% ont ajoutés. L'incubation s'effectue pendant 1 heure à température ambiante. Les plaques sont à nouveau lavées 5 fois en PBS TWEEN 0,05%.

La révélation s'effectue à l'aide du kit BIORAD (n° 170-6432).

Après 1/2 heure à 1 heure, la réaction est arrêtée en lavant les plaques avec de l'eau. Les plaques sont séchées à l'air.

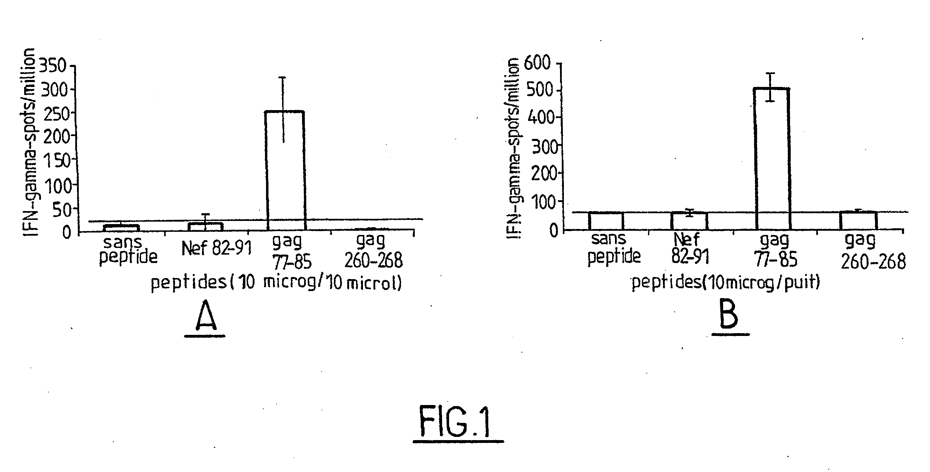

Les résultats sont illustrés par la Figure 1.
A : Essai ELISPOT classique ;
B : Essai effectué avec les peptides fixés sur support solide.

Dans les 2 cas, seul le peptide gag 77-85 induit une activation de lymphocytes T CD8⁺ effecteurs sécrétant de l'IFN-γ. Ces résultats montrent que l'utilisation de peptides fixés sur un support solide est au moins aussi efficace pour induire l'activation de cellules effectrices que celle de peptides présentés en phase liquide.

### EXEMPLE 3 : CARACTERISATION D'UNE IMMUNITE CELLULAIRE VIS-A-VIS DE CMV

Des PBMCs purifiées à partir du sang d'un patient (HLA-A2, B18) ont été testées comme suit.

2 x 10⁵ cellules en suspension dans du milieu classique sont déposés dans chacun des puits dans lesquels lequel ont été fixés les peptides de CMV, comme décrit à l'Exemple 1 ci-dessus.

A titre de témoin positif, un essai a été effectué par la méthode ELISPOT classique : 2 x 10⁵ cellules de suspension des PBMCs du même patient sont déposés dans les puits d'une plaque 96 puits (NUNC) (identique à celle sur laquelle ont été fixés les peptides), et mélangées avec 10 µg de peptide GB 619-628 ou IEI 378-389.

Dans les 2 essais, des PBMCs ont également été déposées dans des puits sans peptides, à titre de contrôle.

L'incubation et la révélation des plaques sont effectuées comme décrit dans l'exemple 2 ci-dessus.

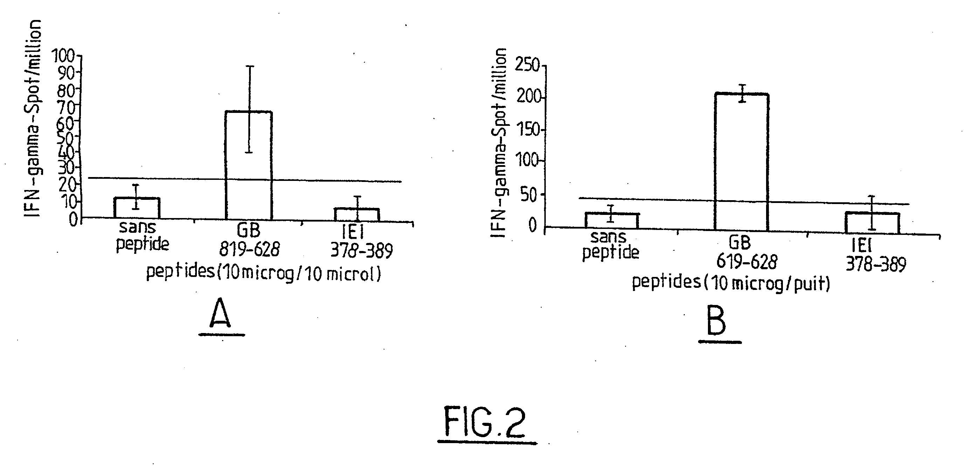

Les résultats sont illustrés par la Figure 2.
A Essai ELISPOT classique ;
B Essai effectué avec les peptides fixés sur support solide.

Dans les 2 cas, seul le peptide GB 619-628 induit une activation de lymphocytes T CD8⁺ effecteurs sécrétant de l'IFN-γ. Ces résultats confirment ceux observés dans le cas des peptides de HIV-1.

### EXEMPLE 4 : CONSERVATION DES PEPTIDES FIXES SUR SUPPORT SOLIDE.

Des plaques dans les puits desquelles ont été fixés les peptides de EBV, comme décrit à l'Exemple 1 ci-dessus, ont été conservées :
A) à 20°C pendant 24H ;
B) à 4°C pendant 7 jours ;
C) à -80°C pendant 15 jours.

2 x 10⁵ PBMCs purifiées à partir du sang d'un patient (HLA B7/B44) en suspension dans du milieu de culture classique sont déposés dans chacun des puits de ces plaques, et testées comme décrit à l'Exemple 3 ci-dessus.

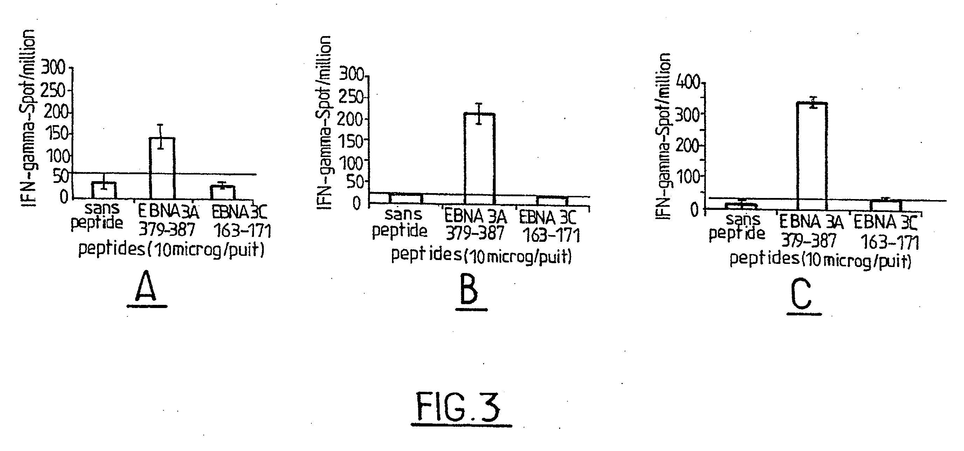

Les résultats sont illustrés par la Figure 3.
A : conservation à 20°C pendant 24H ;
B : conservation à 4°C pendant 7 jours ;
C : conservation à -80°C pendant 15 jours.

Ces résultats montrent que les conditions de conservation ne modifient pas les propriétés d'activation de cellules effectrices des peptides fixés sur support solide.

### EXEMPLE 5 : DETECTION D'UNE IMMUNITE CELLULAIRE VIS-A-VIS DE EBV A L'AIDE D'UN MELANGE DE PEPTIDES FIXES SUR SUPPORT SOLIDE.

Un mélange des peptides suivants :
EBNA 3C 163-171 (EGGVGWRHV, B44),
EBNA 3A 603-611 (RLRAEAGVK, A3),
EBNA 4 416-424 (IVTDFSVIK, A11),
EBNA 3C 881-891 (QPRAPIRPIPT, B7),
EBNA 3A 379-387 (RPPIFIRRL, B7), et
EBNA 6 290-299 (EENLLDFVRF, B44),
constituant des épitopes T CD8⁺ connus de EBV a été fixé sur la paroi interne d'un tube de polystyrène, selon le protocole suivant :

10 µg de chaque peptide dilué en tampon carbonate ont été déposés dans un tube en polystyrène (tube 15 ml, CORNING). La fixation des peptides s'effectue par adsorption pendant 2 heures à température ambiante. Les peptides non-fixés sont éliminés par deux lavages en PBS.

2 ml de sang total d'un patient (HLA : A3-A11, B7/B44) ont été incubés pendant 5 heures dans le tube ainsi obtenu.

Après cette incubation, le sang total a été centrifugé pendant 10 min à 1500 T/min et le surnageant (plasma) du sang a été prélevé. Le plasma (100, 50 et 12,5 microlitres) a été déposé en duplicate sur une plaque de 96 puits pré-incubée avec un anticorps anti-IFN-gamma, pendant 2 h à une température de 20°C. Après lavage, un 2^{ème} anticorps anti-IFN-gamma biotinylé a été ajouté pendant 1 h à une température de 20°C. Puis l'extravidine-PA a été ajoutée pendant 1 h à une température de 20°C et 100 microlitres de substrat MUP (substrat de la phosphase alcaline) a permis la révélation de la réaction. La lecture à été réalisée après 30 min.

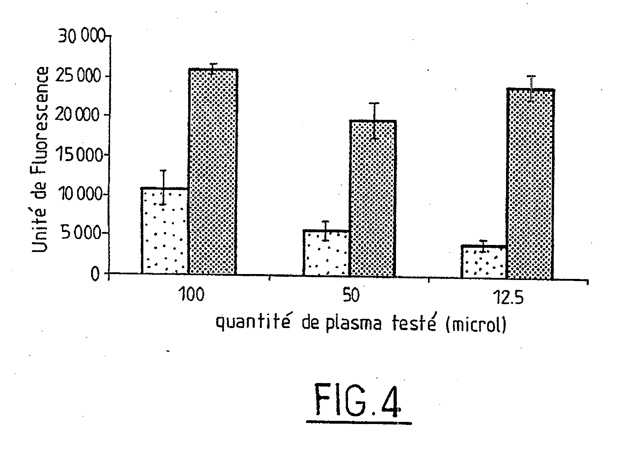

Les résultats sont illustrés par la Figure 4.

Légende de la Figure 4 :
■: sans peptide
■: 10 microgrammes de peptide

Ces résultats montrent qu'il est possible de détecter la présence d'une réponse cellulaire spécifique d'un virus (ici détection du virus EBV) à partir du sang total d'un patient. Cette réponse cellulaire est spécifique au virus et la production d'IFN-gamma a été détectée à différentes dilutions de plasma. Le différentiel est significatif entre la production d'IFN-gamma obtenue à partir du sang total seul (contrôle négatif) ou du sang total incubé avec les peptides du virus EBV. Les cellules présentes dans le sang du patient 1056 ont été activées et ont produit de façon spécifique de l'INF-gamma en présence des peptides CD8⁺ dérivés du virus EBV.

## Revendications

1. Support solide sur lequel est (sont) fixé(s) un ou plusieurs peptide(s) constituant un (des) épitope(s) T CD4+ et/ou T CD8+ d'un antigène.

2. Support solide selon la revendication 1, **caractérisé en ce que** le support est constitué d'un matériau plastique, de nitrocellulose ou d'un matériau à base de silice.

3. Support solide selon la revendication 2, **caractérisé en ce que** le matériau plastique est choisi parmi le groupe constitué par le polystyrène, le polyéthylène ou le chlorure de polyvinyle.

4. Support solide selon la revendication 2, **caractérisé en ce que** le matériau à base de silice est le verre.

5. Support solide selon l'une quelconque des revendications 1 à 4, **caractérisé en ce que** le support solide est choisi parmi le groupe constitué par un tube ou un ensemble de tubes, une plaque de microtitration, une ou plusieurs bandelette(s) ou des billes.

6. Support solide selon l'une quelconque des revendications 1 à 5, **caractérisé en ce que** le ou les peptide(s) est (sont) fixé(s) par adsorption.

7. Support solide selon l'une quelconque des revendications 1 à 5, **caractérisé en ce que** le ou les peptide(s) est (sont) fixé(s) par liaison covalente.

8. Support solide selon l'une quelconque des revendications 1 à 7, **caractérisé en ce que** l'antigène est choisi parmi le groupe constitué par les microorganismes infectieux, les antigènes tumoraux, les antigènes auto-immuns ou les allergènes.

9. Support solide selon la revendication 8, **caractérisé en ce que** les microorganismes infectieux sont choisis parmi le groupe constitué par les virus, les protozoaires ou les bactéries.

10. Support solide selon la revendication 9, **caractérisé en ce que** les virus sont choisis parmi le groupe constitué par les HIV (human immunodeficiency virus) 1 et 2, le FIV (feline immunodeficiency virus), le HTLV-1 (human T lymphocyte virus type I), les virus des différents types d'hépatite ou le cytomégalovirus (CMV).


SEQUENCE LISTING

<110> INSERM
GAHERY-SEGARD, Hanne
GUILLET, Jean-Gérard

<120> TEST DE L'IMMUNITE CELLULAIRE PAR DES
PEPTIDES FIXES SUR SUPPORT SOLIDE

<130> MJPbv598/53

<160> 11

<170> PatentIn version 3.1

<210> 1
<211> 9
<212> PRT
<213> Human immunodeficiency virus type 1

<400> 1
Ser Leu Tyr Asn Thr Val Ala Thr Leu
1               5

<210> 2
<211> 9
<212> PRT
<213> Human immunodeficiency virus type 1

<400> 2
Glu Ile Tyr Lys Arg Trp Ile Ile Leu
1               5

<210> 3
<211> 10
<212> PRT

<213>  Human immunodeficiency virus type 1

<400>  3

Lys Ala Ala Leu Asp Leu Ser His Phe Leu
1               5                   10

<210>  4

<211>  11

<212>  PRT

<213>  Human herpesvirus 4

<400>  4

Phe Ile Ala Gly Asn Ser Ala Tyr Glu Tyr Val
1               5                   10

<210>  5

<211>  12

<212>  PRT

<213>  Human herpesvirus 4

<400>  5

Ser Asp Glu Glu Glu Ala Ile Val Ala Tyr Thr Leu
1               5                   10

<210>  6

<211>  9

<212>  PRT

<213>  Human herpesvirus 5

<400>  6

Arg Pro Pro Ile Phe Ile Arg Arg Leu
1               5

<210>  7

<211>  9

<212>  PRT

<213>  Human herpesvirus 5

<400> 7

Glu Gly Gly Val Gly Trp Arg His Val
1               5

<210> 8

<211> 9

<212> PRT

<213> Human herpesvirus 5

<400> 8

Arg Leu Arg Ala Glu Ala Gly Val Lys
1               5

<210> 9

<211> 9

<212> PRT

<213> Human herpesvirus 5

<400> 9

Ile Val Thr Asp Phe Ser Val Ile Lys
1               5

<210> 10

<211> 11

<212> PRT

<213> Human herpesvirus 5

<400> 10

Gln Pro Arg Ala Pro Ile Arg Pro Ile Pro Thr
1               5                   10

<210> 11

<211> 10

<212> PRT

<213> Human herpesvirus 5

<400> 11

Glu Glu Asn Leu Leu Asp Phe Val Arg Phe
1               5                   10